# EUROPÄISCHE PATENTSCHRIFT

(11) **EP 1 341 819 B1**
(45) Veröffentlichungstag und Bekanntmachung des Hinweises auf die Patenterteilung: **14.06.2006**
(21) Anmeldenummer: 01270546.3
(22) Anmeldetag: 07.12.2001
(51) Int. Cl.: A61K 38/36

(54) **VERWENDUNG VON FIBRIN/FIBRINOGENPEPTIDEN UND -PROTEINEN ZUR HERSTELLUNG EINES ARZNEIMITTELS**
USE OF FIBRIN/FIBRINOGEN PEPTIDES AND PROTEINS FOR THE MANUFACTURE OF A MEDICAMENT
UTILISATION DES PEPTIDES ET PROTEINES DE FIBRINE/FIBRINOGENE DANS LA FABRICATION D'UN MEDICAMENT

(30) Priorität: 12.12.2000 AT 20632000
(43) Veröffentlichungstag der Anmeldung: 10.09.2003
(60) Teilanmeldung: 05012488.2
(73) Patentinhaber: Fibrex Medical Research & Development GmbH, 1010 Wien (AT)
(72) Erfinder: PETZELBAUER, Peter, A-1230 Wien (AT)
(74) Vertreter: Schwarz, Albin
(86) Internationale Anmeldenummer: PCT/AT2001/000387
(87) Internationale Veröffentlichungsnummer: WO 2002/048180

(56) Entgegenhaltungen:
- WO-A-93/21962
- DE-A- 19 729 591
- US-A- 4 927 916
- HARENBERG J ET AL.: "Biodistribution of human fibrinogen-derived peptides in rabits; in: Fibrinogen: Struct. Variants Interact." 1983 , DE GRUYTER , BERLIN (DE) XP008008830 Seite 271 -Seite 278
- IKEMATSU S: "Radioimmunoassay of fibrinopeptide" RINSHO KENSA, Bd. 28, Nr. 1, 1984, Seiten 20-24, XP001115011
- KAWASAKI K ET AL.: "Amino acids and peptides XII: Synthetic peptides related to the N-terminal portion of fibrin alpha-chain and their inhibitory effects on fibrinogen/thrombin clotting" THROMBOSIS RESEARCH, Bd. 56, 1989, Seiten 757-762, XP008008821
- EVERSE SJ ET AL.: "Conformational Changes in Fragments D and Double-D from Human Fibrin(ogen) upon Binding the Peptide Ligand Gly-His-Arg-Pro-Amide" BIOCHEMISTRY, Bd. 38, 18. Februar 1999 (1999-02-18), Seiten 2941-2946, XP002215499
- HERRICK S ET AL.: "Fibrinogen" THE INTERNATIONAL JOURNAL OF BIOCHEMISTRY & CELL BIOLOGY, Bd. 31, 1999, Seiten 741-746, XP002215500
- SKOGEN W.F. ET AL: 'Fibrinogen-Derived Peptide B.beta.1-42 Is a Multidomained Neutrophil Chemoattractant' BLOOD Bd. 71, Nr. 5, Mai 1988, Seiten 1475 - 1479, XP008013244

## Beschreibung

Die Erfindung bezieht sich auf die Verwendung von Peptide und/oder Proteine zur Herstellung eines therapeutischen und/oder präventiven Arzneimittels.

Bisher in Prophylaxe und Therapie verwendete Substanzen zur Hemmung oder Verhinderung von Entzündungsreaktionen, sogenannte. Immunsuppresiva, umfassen im wesentlichen zwei verschiedene Gruppen. Erstens Abkömmlinge eines im Körper natürlich vorkommenden Hormons, dem Cortison und zweitens körperfremde Immunsuppressiva, wie Cyclosporin und deren Derivate, Azathioprin, Cyclophosphamid usw. Diesen Substanzen ist eine antiinflarnmatorische Wirkung gemeinsam, jedoch haben diese Substanzen in der Langzeittherapie erhebliche Nebenwirkungen. Diese Nebenwirkungen limitieren eine langzeitige Therapie, daher werden diese Substanzen alternierend oder in Kombinationen eingesetzt, um Nebenwirkungen auf ein erträgliches Ausmaß zu reduzieren oder um die Therapie überhaupt fortsetzen zu können. Als Nebenwirkung seien die pathologischen Frakturen bei Cortison genannt, die durch den osteoporotischen Effekt des Cortisons verursacht sind oder das Nierenversagen, das durch Cyclosporin hervorgerufen werden kann. Diese Nebenwirkungen sind beiden Verbindungsgruppen obligat, es ist somit lediglich eine Frage der Therapiedauer und Gesamtdosis, wann die Therapie abgesetzt werden muß.

Der vorliegenden Erfindung ist zur Aufgabe gesetzt, neue Pharmazeutika zu schaffen, die geeignet sind, Entzündungsreaktionen zu verhindern oder zu hemmen und lediglich untergeordnete Nebenwirkungen aufweisen. Eine weitere Aufgabe besteht darin, eine Langzeittherapie zur Verfügung zu stellen.

Im folgenden werden die Aminosäuren der erfindungsgemäß verwendeten Peptide mit den üblichen Abkürzungen bezeichnet, wobei diese die α-Aminosäuren bezeichnen.

Die Aufgabe der Erfindung wird gelöst durch die Verwendung von Peptiden oder Proteinen der allgemeinen Formel 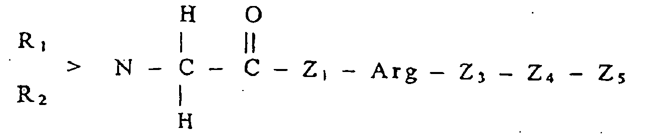 worin R₁ und R₂ gleich oder unterschiedlich, Wasserstoff, einen gesättigten oder ungesättigten Kohlenwasserstoffrest mit 1 bis 3, insbesondere bis 10, Kohlenstoffatomen, worin
a) Z₅ einen Peptidrest mit folgender Aminosäuresequenz (SEQ ID NO 11): und
   - Z₁: Histidinrest
   - Arg: Argininrest
   - Z₃: Prolinrest
   - Z₄: Leucinrest
   bedeuten,
   oder worin
b) Z₅ einen Peptidrest mit folgender Aminosäuresequenz (SEQ ID NO 12): und
   - Z₁: Prolinrest
   - Arg: Argininrest
   - Z₃: Valinrest
   - Z₄: Valinrest
   bedeuten,
   sowie deren Salze, als auch z.B. Amide, oder Stoffgemische dieser miteinander und/oder zumindest einem weiteren Stoff zur Herstellung eines Arzneimittels zu einer therapeutischen und/oder präventiven Verwendung in der Human- und/oder Veterinärmedizin zur Verhinderung der Adhäsion von Zellen aus dem Blutstrom an Endothelzellen der Gefäßwand und/oder einer nachfolgenden Transmigration aus dem Blut in ein Gewebe.

Es ist bekannt, daß Fibrinogen die beiden Peptidketten Aalpha und Bbeta aufweist und daß bei entzündlichen Prozessen die Synthese von Fibrinogen stark zunimmt (Herrick et al., The International Journal of Biochemistry & Cell Biology, Bd. 31, 1999, S. 741-746). Ferner wird das Peptid Bβ₁₅₋₄₂ erwähnt (Ikematsu, Rinsho Kensa, Bd. 28, Nr. 1, 1984, S. 20-24). Aus Thrombosis Research 56, 1989, S. 757-762 sind verschiedenste Tri- und Tetrapeptide bekannt, die anti-thrombotische Wirksamkeit aufweisen. Die physiologische Rolle von Fibrinogen-Spaltprodukten wird auch in Blood, Bd. 71, 1988, S. 1475-1479, angesprochen.

Es war vollkommen überraschend, daß die oben definierte Aminosäuresequenz die Adhäsion von Zellen aus dem Blutstrom an Endothelzellen der Gefäßwand und/oder ihre nachfolgende Transmigration aus dem Blut in das Gewebe verhindern.

Es war ferner vollkommen überraschend, daß S equenzteile, Peptide oder Bruchstücke des Fibrinogens eine antiinflammatorische Wirkung aufweisen. Ohne durch diese theoretischen Überlegungen gebunden zu sein, könnte diese Wirkung darauf beruhen, daß das Fibrin über seinen neo-N-Terminus der Bbeta-Kette an Endothelzellen und über die Sequenz der Aalpha-Kette an Zellen im Blutstrom bindet und so zur Adhäsion und d Transmigration von Zellen ins Gewebe führt. Diese Verbindungen haben eine Nebenwirkung, und zwar wird die Fibrinbildung gehemmt. Diese Hemmung bedeutet jedoch für den Patienten keinen potentiellen Nachteil, da die Blutgerinnung auch in Abwesenheit von Fibrin bei banalen Verletzungen ausreichend ist. Lediglich bei chirurgischen Eingriffen könnte gegebenenfalls ein Absetzen einer derartigen Therapie zweckmäßig sein. Andere Nebenwirkungen sind im wesentlichen auszuschließen, da diese Substanzen nur mit den natürlichen Liganden interagieren. Weiters wird die natürliche Abwehr durch die Leukozyten im Blut nicht negativ beieinflußt. So bleibt die Zusammensetzung derselben, wie Granulozyten, Lymphozyten und Monozyten unbeeinflußt, so daß der natürliche Abwehrprozeß erhalten bleibt und die Infektabwehr im Blut unverändert bleibt.

Fibrinogen wird .in der Leb.er gebildet und ist in dieser Form biologisch inaktiv und befindet sich normalerweise in Konzentrationen um 3 g/l im Blut. Durch proteolitisch.e Spaltung des Proenzyms Prothrombin wird Thrombin gebildet, welches vom Fibrinogen die Fibrinopeptide A und B abspaltet. Dadurch wird Fibrinogen in seine biologisch aktive Form umgewandelt. Es entstehen Fibrin und Fibrinspaltprodukte.

Thrombin wird bei jeder Aktivierung der Blutgerinnung gebildet, also bei jedem Gewebeschaden, sei dieser entzündlicher, traumatischer oder degenerativer Genese. Die durch Thrombin mediierte Bildung von Fibrin ist prinzipiell ein protektiver Vorgang, um entstandene Defekte im Gefäßsystem rasch abzudichten. Die Bildung von Fibrin ist jedoch auch ein pathogener Vorgang. Die Entstehung eines Fibrinthrombus als auslösende Ursache beim Herzinfarkt ist einer der prominentesten Probleme in der Humanmedizin. Bisher nicht oder nicht ausreichend untersucht wurde die Rolle von Fibrin bei der Extravasation von Entzündungszellen aus dem Blutstrom ins Gewebe, ein einerseits erwünschter Vorgang bei der Abwehr von pathogenen Mikroorganismen oder Tumorzellen im Gewebe, andererseits aber ein Vorgang, der durch sich selbst Gewebeschaden induziert oder weiter erhält. Fibrin bindet über seinen neo-N-Terminus der Bbeta an Endothelzellen mittels der Sequenz zu Bbeta und an Zellen im Blutstrom mittels der Sequenz Aalpha und führt so zur Adhäsion und Transmigration von Zellen ins Gewebe.

Die erfindungsgemäß Verwendeten Peptide oder Proteine können die Adhäsion von-Zellen aus dem Blutstrom an Endothelzellen der Gefäßwand und/oder ihre nachfolgende Transmigration aus dem Blut ins Gewebe verhindern.

Ein erfindungsgemäß Verwendetes Peptid oder Protein, worin Z₅ ein Peptidrest mit folgender Aminosäuresequenz (SEQ ID NO 11): und
- Z₁: Histidinrest
- Arg: Argininrest
- Z₃: Prolinrest
- Z₄: Leucinrest
bedeuten, verhindert eine Ablagerung bzw. Anheftung von Fibrinbruchstücken an die Gefäßwand. Damit können Entzündungszellen an Endothelzellen der Gefäßwandung von Arterien und Venen nicht festgehalten werden und es wird verhindert, daß derartige Zellen an der Gefäßwandung verbleiben und so weiter in das Gewebe gelangen können.

Ein erfindungsgemäß verwendetes Peptid oder Protein, worin Z₅ ein Peptidrest mit folgender Aminisäuresequenz (SEQ ID NO 12): und
- Z₁: Prolinrest
- Arg: Argininrest
- Z₃: Valinrest
- Z₄: Valinrest

bedeuten, bewirkt, daß Zellen des periphären Blutes nicht an Fibrin oder Fibrinbruchstücken anhaften können und damit wird deren Migration im Gewebe verhindert.

Die beschriebenen Spaltprodukte sind auch in der Literatur als Peptid Bbeta und Peptid Aalpha bekannt. Dieser oben angeführte proadhäsive und promigratorische Weg ist ein völlig neuer für das System der Steuerung der Wanderung von Zellen aus dem Blut in das Gewebe. Diese Funktion von Fibrin kann sowohl durch das Peptid Bbeta als auch durch das Peptid Aalphablockiert werden.

Diese erfindungsgemäß Verwendeten Peptide bieten sich daher als Therapeutikum bei Mensch und Tier an, um die Wanderung von Zellen aus dem Blut ins Gewebe zu blockieren. Da Fibrin oder andere Produkte des Fibrinogens, die durch proteolytische Spaltung entstehen, wie z. B. Urokinase-Plasminogen-Aktivator gespaltenes Fibrinogen nur spezifisch und regional begrenzt entstehen, also an Stellen von Entzündung, Gerinnungsstörung, Arteriosklerose, Thrombose und/oder Tumorwachstum, handelt es sich hierbei um ein regional beschränkt wirksames Therapeutikum, also pathalogische Nebenwirkungen an anderen Orten sind nicht oder nur in beschränktem Ausmaß zu erwarten.

Völlig unerwartete Anwendungsbereiche der erfindungsgemäßen Peptide und/oder Proteine liegen daher in der Herstellung von Arzneimitteln zur Therapie oder Prävention von lokalen und/oder generalisierten Entzündungen des Körpers bei infektiöser Genese, auf Basis einer Autoimmunreaktion, auf Basis einer rheumatischen Erkrankung, auf Basis einer Störung des Immunsystems, auf Basis einer genetischen Erkrankung, zur Prävention und/oder Therapie der Abstoßungsreaktion bei Organtransplantationen, der Arteriosklerose, des Reperfusionstraumas, auf Basis arteriosklerotischer und/oder thrombotischer Erkrankungen und vermehrter Fibrindeposition vor. Ein derartiges Peptid, insbesondere Bbeta, ist auch zur Herstellung eines Arzneimittels hervorragend geeignet, das den Transport eines weiteren Arzneimittels an menschliche oder tierische Endothelzellen bewerkstelligt. Hierbei wird das zu transportierende Arzneimittel an einem Ende mit dem Peptid gekoppelt und lagert sich sodann an einer freien Stelle der Gefäßwandung, u. zw. an einer Endothelzelle, über VE-Cadherin an.

Im folgenden wird die Erfindung anhand der Beipiele näher erläutert.

### Beispiel 1:

### Herstellung der Fibrinogenspal-tprodukte:

Nicht polymerisierende Degradationsprodukte von Fibrinogen wurden durch Abbau mit Cyanogenbromid gemäß Blombzck et al. (Nature 1968, 218; 130-134) gewonnen. Das so abgebaute Fibrinogen besteht zum Großteil aus einem 63 kD Fragment, und zwar dem N-terminalen Disulfid Knoten, NDSK, und beinhaltet die Aalpha Kette 1-51, Bbcta Kette 1-118 und gamma Kette 1-78. Um NDSK-II zu erhalten (NDSK minus Fibrinopeptide A und B), wurden die N-terminalen Aminosäuren der Aalpha und Bbeta Kette mit Thrombin (20 Einheiten/1 µg NDSK) in drei Stunden bei Raumtemperatur abgespalten und nachfolgend mit Diisopropylfluorophosphat zur Blockierung der Thrombinaktivität behandelt. Das so erhaltene NDSK-II bestand aus Aalpha Kette 17-51, Bbeta Kette 15-118 und gamma Kette 1-78.

Um NDSK-uPA zu erhalten, wurden 500 µg NDSK eine Stunde bei 37°C mit 200 Einheiten Urokinase-Plasminogen-Aktivator (uPA) der Firma Technoclone, Wien, Österreich, behandelt. Die Reaktion wurde mit 5 mM Phenylmethylsulfonylfluorid abgebrochen. Das so erhaltene NDSK-uPA ist ein NDSK und weist kein Fibrinopeptid B auf.

Für Negativkontrollen wurde eine zweite Fraktion aus den durch Cyanogenbromid Behandlung entstandenen Fibrinogenspaltprodukten, genannt FCB-2 gemäß Nieuwenhuizen et al. (Biochem Biophys Acta 1983, 755; 531 - 533) gewonnen. FCB-2 ist ein 43 kD großes Protein und besteht aus der Aalpha Kette 148-208, der Bbeta Kette 191-305 und der gamma Kette 95-265. Dieses Protein wurde zu Kontrollzwecken ebenso mit Thrombin und mit Diisopropylfluorophosphat versetzt. Es führte jedoch zu keiner Änderung des Proteins (im folgenden FCB-2-thr genannt).

Für weitere Negativkontrollen wurde Kulturmedium (RPMI der Firma Life techn. Inc., Paisky, UK) mit Thrombin, wie oben behandelt und nachfolgend inaktiviert (RPMI-thr) oder mit uPA, wie oben behandelt und inaktiviert (RPMI-uPA).

### Beispiel 2:

Das Peptid Aalpha (SEQ ID NO 12) entspricht den Aminosäuren 1 bis 28 und mit der Sequenz der alpha Kette des Fibrins und ist ident den Aminosäuren 17 bis 45 der Sequenz der Aalpha Kette des Fibrinogens:

Das Peptid Bbeta (SEQ ID NO 11) entspricht den Aminosäuren 1 bis 28 und mit der Sequenz der beta Kette des Fibrins, das ident mit den Aminosäuren 15 bis 43 der Sequenz der Bbeta Kette des Fibrinogens ist, das folgende Sequenz aufweist:

Beide Peptide wurden mittels Festphasen-Peptidsynthese gemäß Merrifield R. B., J. Amer. Chem. Soc. 1963; 85, 2149-2154 mit einem multiplen Peptidsynthesizer unter Anwendung einer Fluorenylmethyloxycarbonyl (FMOC)-Schutzgruppenstrategie gemäß Carpino L. A. und Han. G Y, J. Amer. Chem. Soc. 1981; 37; 3404-3409 synthetisiert. Die Reinigung der Rohpeptide erfolgte mittels präparativer reversed-phase HPLC über eine Nucleosil 100-10, C18 Säule gemäß Engelhart H. und Müller H. Chromatography 1984 19:77 sowie Henschen A., Hupe K. P. und Lottspeich F. High Performance Liquid Chromatography VCH 1985. Als Kontrollpeptide wurden Peptide mit derselben Länge aber mit randomisierter Aminosäuresequenz verwendet.

### Beispiel 3:

### HU-SCID Maus Modell:

Humane Haut wurde auf den Rücken von SCID Mäusen transplantiert und zwei Wochen danach humane Lymphozyten in das Peritoneum injiziert. Es wurde gemäß Petzelbauer et al. (J. Invest. Dermatol. 1996, 107; 576 - 581) verfahren. Es wurde sodann jeweils fünfzehn derartig präparierten Mäusen in die Schwanzvene injiziert:
a) 100 µg humanes NDSK-II
b) 100 µg humanes FCB-2
c) 100 µg Peptid Aalpha
d) 100 µg Peptid Bbeta
e) 100 µg randomisiertes Aalpha
f) 100 µg randomisiertes Bbeta

Vierundzwanzig Stunden danach wurde die humane Haut entnommen und die Anzahl der Entzündungssstellen, ausgedrückt in Zellen pro 0,3 mm², ermittelt und das Mittel mit Standardabweichung bestimmt.

| | |
|---|---|
| Bei a: | 22 +/- 2,8 |
| bei b: | 9 +/- 2,1 |
| bei c: | 4 +/- 1,1 |
| bei d: | 6 +/- 1,1 |
| bei e: | 5 +/- 1,2 |
| bei f: | 7 +/- 1,3 |

Daraus ist abzuleiten, daß NDSK-II zu Entzündungen führt, und es wurde dieses Protein infolge als pathogene Substanz eingesetzt. Die anderen Verbindungen zeigen für sich keine signifikante Erhöhung der Entzündungszellen.

### Vergleichsbeispiel 4:

Fünfzehn Mäusen gemäß Beispiel 3 wurden

| | |
|---|---|
| 100 µg | humanes NDSK-II und |
| 100 µg | randomisiertes Peptid Aalpha |

in die Schwanzvene injiziert. Es wurde gemäß Beispiel 3 weiterverfahren. Es konnten 23 +/- 3,5 Entzündungsstellen pro 0,3 mm² ermittelt werden.

### Vergleichsbeispiel 5:

Fünfzehn Mäusen gemäß Beispiel 3 wurden

| | |
|---|---|
| 100 µg | humanes NDSK-II gemäß Beispiel 1 und |
| 100 µg | randomisiertes Peptid Bbeta |

in die Schwanzvene injiziert. Es wurde gemäß Beispiel 3 weiterverfahren. Es konnten 24 +/- 2 Entzündungsstellen pro 0,3 mm² ermittelt werden.

### Beispiel 6:

### Fünfzehn Mäusen gemäß Beispiel 3 wurden

| | |
|---|---|
| 100 µg | humanes NDSK-II und |
| 100 µg | synthetisiertes Peptid Aalpha |

injiziert. Es wurde gemäß Beispiel 3 weiterverfahren. Es konnten 21 +/- 2,2 Entzündungsstellen pro 0,3 mm² ermittelt werden.

### Beispiel 7:

Fünfzehn Mäusen gemäß Beispiel 3 wurden

| | |
|---|---|
| 100 µg | humanes NDSK-II und |
| 100 µg | synthetisiertes Peptid Bbeta |

in die Schwanzvene injiziert. Es wurde gemäß Beispiel 3 weiterverfahren. Es konnten 14 +/- 2 Entzündungsstellen pro 0,3 mm² ermittelt werden.

Den Beispielen 4 bis 7 ist zu entnehmen, daß das Peptid Bbeta die lymphozytäre Entzündung blockiert.

### Vergleichsbeispiel 8:

Humane umbilikale Venen Endothelzellen (HUVEC) wurden mit rotem Floureszenzfarbstoff markiert (Cell Tracker Orange, 1 µl/ml, Molecular Probes, Eugene, OR) und auf einer Kollagenmatrix (Collaborative Biomedical Products, Bedford, MA) ausgesät. Nach Konfluenz der Endothelzellen wurden mit grünem Floureszenzfarbstoff (Cell Tracker Green, 1 µl/ml, Molecular Probes der Firma Eugene Origon) markierte periphere Blut mononucleare Zellen (PBMC) (10⁵ Zellen pro 25 mm²) überschichtet. Danach wurden die Zellen für zwölf Stunden auf 37°C inkubiert.

Adhärierende und ins Gel transmigrierte Zellen wurden mit einem Laserscanmikroskop fotografiert, in Pixel transformiert und mittels "NIH image" gemäß Gröger et al. (J. Immunol. Method 1999; 222: 101-109) ausgewertet.

Die Anzahl der adhärenten Zellen pro 0,1 mm² konnte, wie unter Adhäsion angeführt, ermittelt werden. Die Anzahl der migrierten Zellen pro 0,04 mm³ konnte, wie unter Migration angeführt, ermittelt werden. Es wurde der Mittelwert von dreimal drei Versuchen gemeinsam mit der Standardabweichung bestimmt.

| | | Adhäsion | Migration |
|---|---|---|---|
| a) RPMI-uPA | 0,1 1 µg/ml | 40 +/- 4 | 4 +/- 3 |
| | 1,0 µg/ml | 38 +/- 2 | 5 +/- 2 |
| | 10,0 µg/ml | 32 +/- 4 | 5 +/- 1 |
| | | | |
| b) NDSK | 0,1 1 µg/ml | 31 +/-18 | 6 +/- 3 |
| | 1,0 µg/ml | 35 +/- 18 | 5 +/- 2 |
| | 10,0 µg/ml | 36 +/-24 | 6 +/- 3 |
| | | | |
| c) NDSK-II | 0,1 µg/ml | 55 +/-21 | 12 +/- 5 |
| | 1,0 µg/ml | 67 +/-31 | 19 +/-12 |
| | 10,0 µg/ml | 65 +/-31 | 19 +/- 1 0 |
| | | | |
| d) NDSK-uPA | 0,1 µg/ml | 58 +/- 3 | 10 +/- 2 |
| | 1,0 µg/ml | 60 +/- 3,5 | 14 +/- 3 |
| | 10,0 µg/ml | 65 +/- 3 | 18 +/- 1,5 |
| | | | |
| e) FCB2 | 0,1 µg/ml | 30 +/-26 | 6 +/- 4 |
| | 1,0 µg/ml | 10 +/- 10 | 3 +/- 2 |
| | 10,0 µg/ml | 21 +/- 7 | 5 +/- 4 |
| | | | |
| f) FCB-2-thr | ü, 1 µg/ml | 20 +/- 12 | 6 +/- 5 |
| | 1,0 µg/ml | 23 +/- 13 | 7 +/- 5 |
| | 10,0 µg/ml | 26 +/- 11 | 4 +/- 2 |
| | | | |
| g) RPMI-thr | 0,1 µg/ml | 29 +/- 15 | 4 +/- 5 |
| | 1,0 µg/ml | 26 +/- 14 | 5 +/- 5 |
| | 10,0 µg/ml | 41 +/-20 | 5 +/- 4 |

Daraus ist abzuleiten, daß NDSK-II mehr als NDSK-uPA zu signifikanten Migrationen von peripheren Blut-monozellulären Zellen (PBMC) führt und daher pathogen wirkt. Keine der Kontrollen a), b), e), f) und g) führten zu einer signifikanten Migration.

### Beispiel 9:

Die Kollagenmatrix gemäß Beispiel 8 mit der Suspension aus PBMC wurde mit 100 µg NDSK-II und Bbeta oder Bbeta randomisiert versetzt, und es wurde gemäß Beispiel 8 weiterverfahren.

| | | Adhäsion | Migration |
|---|---|---|---|
| a) | kein Zusatz von NDSK-II | 38 +/- 15 | 6 +/- 4 |
| b) | nur 100 µg NDSK-II | 73 +/-29 | 16 +/-7 |
| c) | 10 µg Bbeta + NDSK-II | 63 +/-33 | 7 +/-4 |
| d) | 100 µg Bbeta + NDSK-II | 47 +/-34 | 5 +/-4 |
| e) | 1000 µg Bbeta + NDSK-II | 52 +/-27 | 10 +/- 6 |
| f) | 10 µg Bbeta randomisiert + NDSK-II | 77 +/-33 | 16 +/- 6 |
| g) | 100 µg Bbeta randomisiert + NDSK-II | 86 +/-35 | 15 +/-6 |
| h) | 1000 µg Bbeta randomisiert + NDSK-II | 78 +/-31 | 13 +/- 8 |

Wie diesen Versuchsergebnissen zu entnehmen, blockiert das Peptid Bbeta Entzündungen.

### Beispiel 10:

Die Kollagenmatrix gemäß Beispiel 8 mit der Suspension aus PBMC wurde mit 100 µg NDSK-11 und Aalpha oder Aalpha randomisiert versetzt, und es wurde gemäß Beispiel 8 weiterverfahren.

| | | Adhäsion | Migration |
|---|---|---|---|
| a) | kein Zusatz von NDSK-11 | 42 +/- 6 | 10 +/- 1 |
| b) | nur NDSK-II | 96 +/- 11 1 | 24 +/- 3 |
| c) | 10 µg Aalpha + NDSK-II | 69 +/- 12 | 21 +/- 4 |
| d) | 100 µg Aalpha + NDSK-11 | 73 +/- 13 | 15 +/- 6 |
| e) | 1000 µg Aalpha + NDSK-II | 70 +/- 6 | 13 +/- 5 |
| f) | 10 µg Aalpha randomisiert + NDSK-II | 70 +/- 6 | 25 +/- 2 |
| g) | 100 µg Aalpha randomisiert + NDSK-II | 65 +/- 16 | 24 +/- 3 |
| h) | 1000 µg Aalpha randomisiert + NDSK-II | 70 +/- 12 | 26 +/- 3 |

Aus den Versuchsergebnissen läßt sich ableiten, daß das Peptid Aalpha nur teilweise die Migration von PBMC blockiert.

### Beispiel 11:

Da PBMC im wesentlichen aus einem Gemisch von Lymphozyten und Monozyten besteht, wurden im Beispiel 11 reine Lymphozyten anstelle von PBMC (wie in den Beispielen 8 - 10) verwendet.

Die Kollagenmatrix gemäß Beispiel 8 mit Endothelzellen und Lymphzyten wurde mit 100 µg NDSK-uPA bzw. 100 µg NDSK-II und Aalpha bzw. Bbeta versetzt.

| | | Adhäsion | Migration |
|---|---|---|---|
| a) | kein Zusatz | 68 +/- 8 | 16 +/- 3 |
| b) | NDSK-uPA | 143 +/-11 | 53 +/- 5 |
| c) | NDSK-II | 119 +/-11 | 43 +/- 4 |
| d) | nur 100 µg Bbeta | 58 +/- 18 | 14 +/- 1 |
| e) | NDSK-uPA + 100 µg Bbeta | 74 +/- 8 | 19 +/- 2 |
| f) | NDSK-II + 1 00 µg Bbeta | 74 +/- 8 | 17 +/- 3 |
| g) | nur 100 µg Aalpha | 77 +/- 4 | 18 +/-1 |
| h) | NDSK-uPA + 100 µg Aalpha | 131 +/- 4 | 40 +/- 3 |
| i) | NDSK-II + 100 µg Aalpha | 131 +/- 4 | 44 +/- 4 |
| j) | nur 100 µg Bbeta randomisiert | 75 +/- 5 | 19 +/- 1 |
| k) | NDSK-uPA + 100 µg Bbeta randomisiert | 134 +/- 13 | 46 +/-4 |
| 1) | NDSK-II + 100 µg Bbeta randomisiert | 120 +/- 12 | 42 +/- 4 |

Aus diesen Versuchsergebnissen folgt:
1) daß sowohl NDSK-II als auch NDSK-uPA die Lymphozyten Entzündung fördern,
2) daß das Peptid Bbeta die durch NDSK-II und NDSK-uPA induzierte Lymphozyten Adhäsion und Migration zur Gänze blockiert, hingegen das Peptid Aalpha keinen blockierenden Effekt hat, woraus anzunehmen ist, daß die freie Alpha Kette zur Induktion der Adhäsion und Migration der Lymphozyten nicht benötigt wird.

### Beispiel 12:

Es wurde gemäß Beispiel 11 verfahren, jedoch anstelle der Lymphozyten wurden reine Monozyten verwendet. Es wurde 100 µg NDSK-uPA bzw. 100 µg NDSK-II mit Peptid Aalpha, randomisiertem Aalpha, Bbeta oder randomisierte, Bbeta versetzt.

| | | Adhäsion | Migration |
|---|---|---|---|
| a) | kein Zusatz | 43 +/- 8 | 7 +/- 1 |
| b) | NDSK-uPA | 48 +/- 10 | 10 +/- 2 |
| c) | NDSK-II | 90 +/- 11 | 19 +/- 6 |
| d) | 100 µg Bbeta | 59 +/- 7 | 5 +/- 1 |
| e) | NDSK-uPA + 100 µg Bbeta | 61 +/- 1 1 | 8 +/- 3 |
| f) | NDSK-II + 100 µg Bbeta | 70 +/- 7 | 7 +/- 5 |
| g) | 100 µg Bbeta randomisiert | 40 +/- 7 | 6 +/- 1 |
| h) | NDSK-uPA + 100 µg Bbeta randomisiert | 45 +/- 5 | 8 +/- 3 |
| g) | NDSK-II + 100 µg Bbeta randomisiert | 92 +/-10 | 20 +/- 7 |
| j) | 100 µg Aalpha | 59 +/- 6 | 5 +/-1 |
| k) | NDSK-uPA + 100 µg Aalpha | 62 +/- 4 | 8 +/- 5 |
| 1) | NDSK-II + 100 µg Aalpha | 68 +/- 10 | 9 +/- 6 |
| m) | 100 µg Aalpha randomisiert | 58 +/- 7 | 6 +/- 1 |
| n) | NDSK-uPA + 100 µg Aalpha randomisiert | 50 +/- 10 | 10 +/- 4 |
| o) | NDSK-II + 100 µg Aalpha randomisiert | 108 +/- 8 | 21 +/- 5 |

Aus diesen Versuchsergebnissen folgt, daß nur NDSK-II und nicht NDSK-uPA die Wanderung von Monozyten fördert, also sowohl Alpha Kette als auch die Beta Kette ein freies N-terminales Ende aufweisen müssen und die Wanderung der Monozyten blockieren.

### Beispiel 13:

Es wurde gemäß Beispiel 11 verfahren, es wurden reine Lymphozyten verwendet. Es wurde 100 µg NDSK-uPA bzw. 100 µg NDSK-II mit den kurzen Peptidsalzen abgeleitet von Aalpha Gly Pro Arg (Pro)-NH₂ acetat (Aalpha-derivat) oder abgeleitet von Bbeta Gly His Arg Pro-OH acetat (Bbeta-derivat) versetzt.

| | | Adhäsion | Migration |
|---|---|---|---|
| a) | kein Zusatz | 60 +/- 8 | 14 +/- 1 |
| b) | NDSK-uPA | 149 +/- 12 | 57 +/- 5 |
| c) | NDSK-II | 121 +/- 11 | 48 +/- 7 |
| d) | nur 100 µg Bbeta-derivat | 58 +/- 10 | 12 +/- 9 |
| e) | NDSK-uPA + 100 µg Bbeta-derivat | 70 +/- 8 | 16 +/- 3 |
| f) | NDSK-II + 100 µg Bbeta-derivat | 69 +/- 7 | 14 +/- 5 |
| g) | nur 100 µg Aalpha-derivat | 77 +/- 4 | 18 +/-1 |
| h) | NDSK-uPA + 100 µg Aalpha-derivat | 134 +/- 4 | 48 +/- 5 |
| i) | NDSK-II + 100 µg Aalpha-derivat | 131 +/- 7 | 49 +/- 6 |
| j) | nur 100 µg Bbeta-derivat randomisiert | 70 +/- 5 | 14 +/- 7 |
| k) | NDSK-uPA + 100 µg Bbeta-derivat randomisiert | 130 +/- 12 | 49 +/- 6 |
| 1) | NDSK-II + 100 µg Bbeta-derivat randomisiert | 120 +/- 1 0 | 55 +/- 8 |

Aus diesem Experiment ergibt sich, daß bei Hemmung der Lymphozytenmigration diese kurzen Peptide in entsprechender kontinuierlicher Zugabe gleich wirksam wie die langen Peptide sind.

### Beispiel 14:

Es wurde gemäß Beispiel 12 verfahren, es wurden reine Monozyten verwendet. Es wurde 100 Mg NDSK-uPA bzw. 100 µg NDSK-II mit den kurzen Peptidsalzen Aalpha Gly Pro Arg (Pro)-NH₂ acetat (Aalpha-derivat) oder Bbeta Gly His Arg Pro-OH acetat (Bbeta-derivat) versetzt.

| | | Adhäsion | Migration |
|---|---|---|---|
| a) | kein Zusatz | 40 +/- 8 | 5 +/- 1 |
| b) | NDSK-uPA | 54 +/- 9 | 7 +/- 2 |
| c) | NDSK-II | 85 +/- 1 1 | 22 +/- 6 |
| d) | 100 µg Bbeta-derivat | 52 +/- 7 | 6 +/- 1 |
| e) | NDSK-uPA + 100 µg Bbeta-derivat | 61 +/- 1 1 | 8 +/- 3 |
| f) | NDSK-II + 100 µg Bbeta-derivat | 68 +/- 7 | 8 +/- 4 |
| g) | 100 µg Bbeta-derivat randomisiert | 40 +/- 7 | 6 +/- 1 |
| h) | NDSK-uPA + 100 µg Bbeta-derivat randomisiert | 44 +/- 6 | 8 +/- 2 |
| i) | NDSK-II + 100 µg Bbeta-derivat randomisiert | 92 +/- 10 | 23 +/- 7 |
| j) | 100 µg Aalpha-derivat | 50 +/- 5 | 4 +/- 4 |
| k) | NDSK-uPA + 100 µg Aalpha-derivat | 60 +/- 5 | 7 +/- 6 |
| 1) | NDSK-II + 100 µg Alpha-derivat | 64 +/- 11 | 8 +/- 2 |
| m) | 100 µg Aalpha-derivat randomisiert | 54 +/- 1 0 | 6 +/- 3 |
| n) | NDSK-uPA + 100 µg Aalpha-derivat randomisiert | 50 +/- 10 | 10 +/- 4 |
| o) | NDSK-II + 100 µg Aalpha-derivat randomisiert | 99 +/- 8 | 21 +/- 7 |

Aus diesem Experiment ergibt sich, daß bei Hemmung der Monozytenmigration diese kurzen Peptide in entsprechender kontinuierlicher Zugabe gleich wirksam wie die langen Peptide sind.

### Beispiel 15:

Die Versuche wurden mit männlichen Wistarratten mit einem Gewicht zwischen 220 g und 280 g durchgeführt. Die Ratten wurden mit einer Standarddiät und Wasser ernährt. Für die Durchführung des Versuches wurden die Ratten anästisiert und es erfolgte eine künstliche Beatmung mit einer Frequenz von 70 Pulsen pro Minute, wobei 8 ml bis 10 ml pro Kilogramm eines Gases mit 30 Vol.-% Sauerstoff mit einem Überdruck von 1 mm bis 2 mm Quecksilber abgegeben wurde. Die rechte Herzarterie wurde mit einer Meßkanüle versehen, und der Blutdruck in der Arterie als auch die Schläge wurden bestimmt. Die Druckrate wurde bestimmt als Produkt des Blutdruckes in der Arterie und der Herzschlagrate mit de Dimsension mm Quecksilber / Minute / 10³. Die rechte Vene wurde mit einer Meßkanüle für die Dotation der Versuchssubstanzen versehen. Nach der chirurgischen Vorbereitung wurden 2 ml Rattenblut dem Herzen zugeführt. Nach dreißig Minuten wurde die linken Herzarterie verschlossen. Nach weiteren fünfundzwanzig Minuten wurde der Verschluß gelöst, um das ischämische Areal wieder zu durchbluten. Zu diesem Zeitpunkt wurden der Hälfte der Tiere 800 µg/kg Peptid Bbeta bzw. Peptid Bbeta randomisiert intravenös verabreicht und sodann zwei Stunden verstreichen gelassen.

Um zwischen geschädigtem und nicht geschädigtem Gewebe des Herzens zu unterscheiden, wurde dann der linken Herzarterie evans blue dye in einer Konzentration von 2 Gew.-% zugegeben. Das entnommene Herz wurde sodann mit fünf horizontalen Schnitten geteilt, die rechte Venenwand entfernt und die Schnitte mit Triphenyltetratolchlorid (1 Gew.-%) zwanzig Minuten lang bei 37°C behandelt, um zwischen normalem und Infarktgewebe zu unterscheiden. Die Schnitte wurden mit Computer unterstützter Planimetrie ausgewertet.

Durch den Gefäßverschluß waren im Herzen der Vergleichsratten 62,5 % des Herzmuskels bedroht, in den Herzen der Versuchsratten 60 %. In den Vergleichsrattenherzen waren 46 % des bedrohten Gewebes abgestorben, hingegen in den Herzen der Versuchsratten 29 %. Dies entspricht einer 37 %igen Reduktion des abgestorbenen Gewebes (p<0,05).

Die erfindungsgemäß verwendeten, Substanzen sind zur Herstellung eines Arzneimittels sind von besonderer Bedeutung:

Bei einem Arzneimittel zur Therapie von Erkrankungen, die durch gewebsschädigende Wirkung von autoreaktiven Lymphozyten entstehen.

Hierzu zählen Erkrankungen aus dem Formenkreis der Autoimmunität, wie z. B. Kollagenosen, rheumatische Erkrankungen, Psoriasis und post-/parainfektiöse Erkrankungen und Erkrankungen, die durch eine Graft versus Host Reaktion entstehen. Eine heilende Wirkung tritt ein, da dieses Arzneimittel die Wanderung der Lymphozyten ins Gewebe blockiert. Die Lymphozyten bleiben somit im Blutstrom und können keine autoreaktive gewebsschädigende Wirkung erzeugen.

Bei einem Arzneimittel zur Therapie und/oder Prävention von Abstoßungsreaktionen bei Organtransplantationen tritt eine heilende Wirkung ein, da dieses Arzneimittel die Wanderung von Lymphozyten aus dem Blutstrom in das Fremdorgan verhindert und somit das Freindorgan nicht durch autoreaktive Lymphozyten zerstört werden kann.

Bei einem Arzneimittel zur Therapie und/oder Prävention von Arteriosklerose bei Organtransplantationen tritt eine heilende Wirkung ein, da dieses Arzneimittel die Wanderung von Lymphozyten und Monozyten in die Gefäßwand und somit die Aktivierung der Zellen der Gefäßwand unterbindet. Damit wird die Arteriosklerose im Gefolge von Organtransplantationen minimiert oder unterbunden.

Bei einem Arzneimittel zur Therapie und/oder Prävention des Reperfusionstraumas nach chirurgisch oder pharmazeutisch induzierter Wiederdurchblutung, wie z. B. nach Herzinfarkt, Schlaganfall, nach Gefäßoperationen, Bypassoperationen und Organtransplantationen, tritt eine heilende Wirkung ein, da dieses Arzneimittel die Wanderung von Lymphozyten und Monozyten in die Gefäßwand hemmt. Das Reperfusionstrauma entsteht durch Sauerstoffmangel/Azidose der Zellen des Gefäßes während der Wiederdurchblutung und führt zu deren Aktivierung. Dadurch haften Lymphozyten und Monozyten an der Gefäßwand und wandern in diese ein. Die Unterbindung der Anhaftung und Einwanderung von Lymphozyten und Monozyten in die Gefäßwand läßt den Hypoxie/ Azidöse-induzierten Schaden abklingen, ohne daß durch die nachfolgende Entzündungsreaktion ein bleibender Gefäßschaden entsteht.

Bei einem Arzneimittel zur Therapie und/oder Prävention der Arteriosklerose im Gefolge von Stoffwechselerkrankungen oder Alterungsprozessen tritt eine heilende Wirkung ein, da dieses Arzneimittel, die Wanderung von Lymphozyten und Monozyten in die Gefäßwand hemmt und damit die daraus resultierende Progredienz der arteriosklerotischen Plaque hemmt.

Das erfindungsgemäß hergestellte Arzneimittel kann auch zum Transport eines weiteren Arzneimittels eingesetzt werden. Das erfindungsgemäße Arzneimittel bindet spezifisch ein Oberflächenmolekül an Endothelzellen. Damit können daran gekoppelte Arzneimittel an Endothelzellen in hoher Konzentration gebracht werden, ohne daß diese an anderen Stellen Nebenwirkungen erzeugen können. Als Beispiel sei hier die Verwendung von zellteilungshemmenden Substanzen genannt, die spezifisch an Endothelzbllen herangeführt eine antiangiogenetische Wirkung ausüben können. Eine heilende Wirkung tritt hier bei Tumorpatienten ein, da das Tumorwachstum durch eine Verhinderung der Endothelzellproliferation und damit durch eine Verhinderung der Neoangiogenese blockiert wird.

### SEQUENCE LISTING

<110> FIBREX Medical Research & Development GmbH Peczelbauer, Peter
<120> Peptide und/oder Proteine sowie Verwendung desselben zur Herstellung eines therapeutischen und/oder präventiven Arzneimittels
<130> 2760 0 PCT
   <140> PCT/AT 01/00387
   <141> 2001-12-07
   <150> AT A 2063/2000
   <151> 2000-12-12
   <160> 2
   <170> PatentIn version 3.1
<210> 11
   <211> 25
   <212> BRT
<213> Artificial Sequence;
<220>
   <221> source
   <223> /note="entspricht den Aminosäuren 6-28 mit der Sequenz der Bbeta Kette des Fibrins"
<400> 1
<210> 12
   <211> 25
   <212> PRT
   <213> Artificial Sequence
<220>
   <221> source
   <223> /note="entspricht den Aminosäuren 6-28 mit der Sequenz der Aalpha
   Kette des Fibrins"
<400> 2

### SEQUENCE LISTING

<110> FIBREX Medical Research & Development GmbH Petzelbauer, Peter
<120> Peptide und/oder Proteine sowie Verwendung desselben zur Herstellung eines therapeutischen und/oder präventiven Arzneimittels
<130> 2760 PCT
   <140> PCT/AT 01/00387
   <141> 2001-12-07
   <150> AT A 2063/2000
   <151> 2000-12-12
   <160> 2
   <170> Patent In version 3.1
<210> 11
   <211> 25
   <212> PRT
<213> Artificial Sequence;
<220>
   <221> source
   <223> /note="entspricht den Aminosäuren 6-28 mit der Sequenz der Bbeta
   Kette des Fibrins"
<400> 1
<210> 12
   <211> 25
   <212> PRT
   <213> Artificial Sequence
<220>
   <221> source
   <223> /note="entspricht den Aminosäuren 6-28 mit der Sequenz der Aalpha Kette des Fibrins"
<400> 2

### SEQUENCE LISTING

<110> FIBREX Medical Research & Development GmbH Petzelbauer, Peter
<120> Peptide und/oder Proteine sowie Verwendung desselben zur Herstellung eines therapeutischen und/oder präventiven Arzneimittels
<130> 2760 PCT
   <140> PCT/AT 01/00387
   <141> 2001-12-07
   <150> AT A 2063/2000
   <151> 2000-12-12
   <160> 2
   <170> Patent In version 3.1
<210> 11
   <211> 25
   <212> PRT
<213> Artificial Sequence;
<220>
   <221> source
   <223> /note="entspricht den Aminosäuren 6-28 mit der Sequenz der Bbeta Kette des Fibrins"
<400> 1
<210> 12
   <211> 25
   <212> PRT
   <213> Artificial Sequence
<220>
   <221> source
   <223> /note="entspricht den Aminosäuren 6-28 mit der Sequenz der Aalpha Kette des Fibrins"
<400> 2

### SEQUENCE LISTING

<110> FIBREX Medical Research & Development GmbH Petzelbauer, Peter
<120> Peptide und/oder Proteine sowie Verwendung desselben zur Herstellung eines therapeutischen und/oder präventiven Arzneimittels
<130> 2760 PCT
   <140> PCT/AT 01/00387
   <141> 2001-12-07
   <150> AT A 2063/2000
   <151> 2000-12-12
   <160> 2
   <170> PatentIn version 3.1
<210> 1
   <211> 28
   <212> PRT
<213> Artificial Sequence;
<220>
   <221> source
   <223> /note="entspricht den Aminosäuren 1-28 mit der Sequenz der Bbeta Kette des Fibrins"
<400> 1
   Gly His Arg Pro Leu Asp Lys Lys Arg Glu Glu Ala Pro Ser Leu Arg
   1 5 10 15
   Pro Ala Pro Pro Pro Ile Ser Gly Gly Gly Tyr Arg
   20 25
<210> 2
   <211> 28
   <212> PRT
   <213> Artificial Sequence
<220>
   <221> source
   <223> /note="entspricht den Aminosäuren 1-28 mit der Sequenz der Aalpha Kette des Fibrins"
<400> 2
   Gly Pro Arg Val Val Glu Arg His Gln Ser Ala Cys Lys Asp Ser Asp
   1 5 10 15
   Trp Pro Phe Cys Ser Asp Glu Asp Trp Asn Tyr Lys
   20 25

## Patentansprüche

1. Verwendung von Peptiden oder Proteinen der allgemeinen Formel 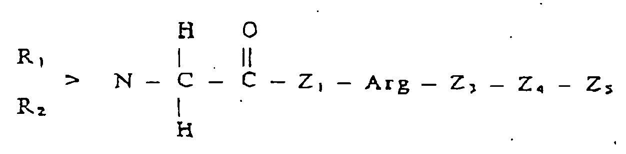 worin R₁ und R₂ gleich oder unterschiedlich, Wasserstoff, einen gesättigten oder ungesättigten Kohlenwasserstoffrest mit 1 bis 3, insbesondere bis 10, Kohlenstoffatomen, worin
a) Z₅ einen Peptidrest mit folgender Aminosäuresequenz (SEQ ID NO 11): und
Z₁ Histidinrest
Arg Argininrest
Z₃ Prolinrest
Z₄ Leucinrest
bedeuten,
oder worin
b) Z₅ einen Peptidrest mit folgender Aminosäuresequenz (SEQ ID NO 12): und
Z₁ Prvlinrest
Arg Argininrest
Z₃ Valinrest
Z₄ Valinrest
bedeuten,
sowie deren Salze, als auch Amide, oder Stoffgemische dieser miteinander und/oder zumindest einem weiteren Stoff zur Herstellung eines Arzneimittels zur Therapie oder Prävention von lokalen und/oder generalisierten Entzündungen des Körpers bei infektiöser Genese, auf Basis einer Autoimmunreaktion, auf Basis einer rheumatischen Erkrankung, auf Basis einer Störung des Immunsystems, auf Basis einer genetischen Erkrankung, zur Prävention und/oder Therapie der Abstoßungsreaktion bei Organtransplantationen, der Arteriosklerose, des Reperfusionstraumas, auf Basis arceriosklerotischer und/oder thrombotischer Erkrankungen und vennehrter Fibrindeposition.

## Claims

1. The use of peptides or proteins of the general formula 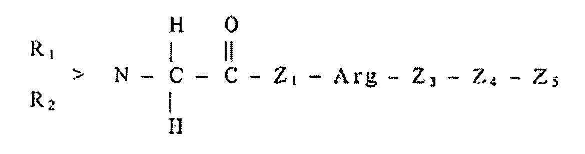
wherein R₁ and R₂, being equal or different, denote hydrogen, a saturated or unsaturated hydrocarbon moiety comprising from 1 to 3, in particular up to 10, carbon atoms, wherein
a) Z₅ denotes a peptide moiety comprising the following amino acid sequence (SEQ ID NO 11): and
Z₁ denotes a histidine moiety
Arg denotes an arginine moiety
Z₃ denotes a proline moiety
Z₄ denotes a leucine moiety
or wherein
b) Z₅ denotes a peptide moiety comprising the following amino acid sequence (SEQ ID NO 12): and
Z₁ denotes a proline moiety,
Arg denotes an arginine moiety,
Z₃ denotes a valine moiety,
Z₄ denotes a valine moiety,
as well as the salts thereof, and also amides, or mixtures of those with each other and/or with at least one further substance for the preparation of a pharmaceutical composition for the therapy or prevention of local and/or generalized inflammations in the body that are of infectious genesis, based upon an auto-immune reaction, based upon a rheumatic disease, based upon a disorder in the immune system, based upon a genetic disease, for the prevention and/or therapy of the rejection occurring after organ transplants, of arterial sclerosis, of a reperfusion trauma, based upon arteriosclerotic and/or thrombotic diseases and increased fibrin deposition.

## Revendications

1. Utilisation de peptides ou de protéines de formule générale 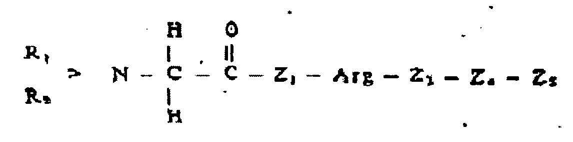 dans laquelle R₁ et R₂ sont identiques ou différents, l'hydrogène, un radical d'hydrogène saturé ou insaturé comportant 1 à 3, en particulier jusqu'à 10 atomes de carbone,
dans laquelle
a) Z₅ est un radical peptidique comportant la séquence d'acides aminés suivante (SEQ ID N° : 11) : et
Z₁ est un radical d'histidine
Arg est un radical d'arginine
Z₃ est un radical de proline
Z₄ est un radical de leucine,
ou dans laquelle
b) Z₅ est un radical peptidique comportant la séquence d'acides aminés suivante (SEQ ID N° : 12) : et
Z₁ est un radical de proline
Arg est un radical d'arginine
Z₃ est un radical de valine
Z₄ est un radical de valine,
ainsi que leurs sels, et également amides ou mélange de ceux-ci les uns avec les autres et/ou au moins une autre substance pour produire un médicament destiné à traiter ou à prévenir les inflammations locales et/ou généralisées d'origine infectieuse de l'organisme, provenant d'une réaction auto-immune, d'une maladie rhumatismale, d'un trouble du système immunitaire, d'une maladie génétique, en prévention et/ou en traitement de la réaction de rejet en cas de greffes d'organes, d'artériosclérose, de lésion de reperfusion, de maladies artérioscléreuses et/ou thrombotiques et de dépôt accru de fibrine.
